# EUROPEAN PATENT SPECIFICATION

(11) **EP 4 384 736 B1**
(45) Date of publication and mention of the grant of the patent: **24.12.2025**
(21) Application number: 22755282.5
(22) Date of filing: 09.08.2022
(51) Int. Cl.: F16K 11/083, F16K 31/56, F16K 31/524, F16K 31/60, F16K 35/04, A61M 16/20

(54) **VALVE**
VENTIL
SOUPAPE

(30) Priority: 10.08.2021 GB 202111500
(43) Date of publication of application: 19.06.2024
(73) Proprietor: Imperial College Innovations Limited, London SW7 2AZ (GB)
(72) Inventor: DHARMADASA, Asela Bandara, London SW7 2AZ (GB); GÓMEZ, Carlos MH, London SW7 2AZ (GB); PATEL, Manish Kumar, London SW7 2AZ (GB); BISHOP, Oliver, Waterlooville PO7 7AN (GB); SMART, Nicholas, Waterlooville PO7 7AN (GB)
(74) Representative: Potter Clarkson
(86) International application number: PCT/GB2022/052074
(87) International publication number: WO 2023/017256

(56) References cited:
- CN-U- 211 536 125
- US-A- 2 702 050
- US-A- 4 150 694
- US-A1- 2002 117 174
- US-A1- 2016 348 795
- US-A1- 2017 059 055

## Description

This disclosure relates to two-way valves for use with breathing assistance equipment and patient ventilator systems.

Critically ill patients often require artificial ventilation to assist or replace spontaneous breathing. This can be provided manually or via a mechanical ventilation system. Mechanical ventilation is termed "invasive" if it involves any instrument penetrating the mouth or the skin. There are two main modes of mechanical ventilation: positive pressure ventilation, where air (or another gas mix) is pushed into the trachea, and negative pressure ventilation, where air is, in essence, sucked into the lungs, e.g. by exerting a sub-atmospheric pressure on the external chest wall.

A patient may need to be temporarily disconnected from a breathing circuit and ventilator for a number of reasons, for example: (i) to change the ventilator e.g. to a portable ventilator for intra- and inter-hospital transfers; (ii) to manually ventilate the patient e.g. with a Water's circuit to improve oxygen saturations; (iii) to change the breathing circuit; (iv) to move or roll the patient.

There can be a number of problems associated with disconnecting a breathing circuit.

A first potential problem relates to staff safety. When a breathing circuit is disconnected, potentially infected aerosols of secretions and water within the breathing circuit can be released into the room (potentially under pressure) and attendant clinical staff or healthcare workers may be sprayed with these aerosols whilst temporarily disconnecting ventilated patients from their breathing circuit.

A second potential problem relates to patient safety. When a breathing circuit is opened to atmosphere, it becomes depressurized and the patient's lungs lose positive end expiratory pressure (PEEP). PEEP is required during ventilation to prevent the collapse of alveoli in the patient's lungs. If alveoli collapse, oxygen levels in the blood may drop and the collapsed alveoli may become more susceptible to infection. It can be difficult to reinflate the alveoli. In patients with severe lung disease, when a breathing circuit is disconnected, it is not uncommon for the oxygen saturations to drop precipitously to dangerous levels within seconds. Historically, the loss of PEEP during breathing circuit disconnection was mitigated by applying a clamp to the endotracheal tube prior to and during disconnection, but this practice may have contraindications. If a patient makes an inspiratory effort whilst a clamp is applied, the negative pressure generated can lead to life threatening complications.

It is an object of the invention to provide an improved method and apparatus for mitigating some or all of the above problems.

US2002117174 (A1) describes a single breath induction anesthesia apparatus for anesthetizing a patient that includes a gas delivery system for delivering at least one gas to the patient, an oxygen supply system for providing oxygen, and an oxygen/anesthesia gas supply system for mixing oxygen and at least one anesthesia gas at a preset optimum ratio sufficient to cause anesthesia of the patient with a single breath, thereby providing an oxygen/anesthesia gas mixture. The apparatus further includes a valve for providing selective gas flow communication between the oxygen supply system and the gas delivery system or between the oxygen/anesthesia gas supply system and the gas delivery system. The valve is operable for first establishing gas flow communication between the oxygen delivery system and the gas delivery system to deliver oxygen to the patient and permit preoxygenation thereof, while inhibiting gas flow communication between the oxygen/anesthesia gas supply system and the gas delivery system to allow the oxygen/anesthesia gas mixture to reach the preset optimum ratio, and thereafter establishing gas flow communication between the oxygen/anesthesia gas supply system and the gas delivery system to deliver the oxygen/anesthesia gas mixture to the patient and permit single breath induction anesthesia thereof, while inhibiting gas flow communication between the oxygen supply system and the gas delivery system. CN211536125 (U) describes a medical three-way joint.

According to a first aspect of the present disclosure there is provided a breathing assistance connector valve comprising:
a valve body defining first, second and third ports;
a valve core defining a ported chamber rotatable about a valve axis between:
   a first stable configuration in which the ported chamber provides fluid communication between the first port and the second port and isolates the third port from the first and second ports; and
   a second stable configuration in which the ported chamber provides fluid communication between the first port and the third port and isolates the second port from the first and third ports,
wherein an outer surface of the valve core is configured to engage with an inner surface of the valve body to define a detent for resisting rotation of the valve core between the first stable configuration and the second stable configuration; and
an actuator configured to overcome the detent to rotate the valve core between the first and second stable configurations.

The actuator may be configured to transition between the two stable configurations and prevent the valve core and valve body from maintaining a stable intermediate position between the first and second stable configurations.

The valve may comprise a biasing element configured to exert a biasing force to urge together the outer surface of the valve core and the inner surface of the valve body. The biasing element may be configured to exert the biasing force along the valve axis. Exerting a biasing force along the valve axis may mean that at least a component of the force is provided along the valve axis, that a substantial component is provided along the valve axis and encompasses the case in which the entire force is aligned with the valve axis. The biasing element may comprise a spring.

The actuator may be configured to facilitate application a rotational force to the valve core about the valve axis.

The actuator may be configured to overcome the detent and the biasing force such that the valve core rotates about the valve axis between the first stable configuration and the second stable configuration. The actuator may be configured to overcome the detent and the biasing force such that the valve core is displaced along the valve axis away from the inner surface of the valve body.

A greater proportion of the inner surface of the valve body and the outer surface of the valve core may be in contact during the first stable configuration and the second stable configuration than during an intermediate position between the first stable configuration and the second stable configuration.

The valve core may comprise a tapered portion defining the ported chamber. A second end of the tapered portion may be narrower than a first end of the tapered portion. The valve body may comprise a tapered portion for receiving the tapered portion of the valve core. The valve core may be substantially frustoconical.

The valve core may comprise one or more sealing portions on an outer surface of the valve core. The one or more sealing portions may be configured to provide a seal between the valve core and the first, second and third ports.

The outer surface of the valve core may be substantially transverse or substantially perpendicular to the valve axis. The inner surface of the valve body may be substantially transverse or substantially perpendicular to the valve axis. The outer surface of the valve core may face the inner surface of the valve body.

The outer surface of the valve core and the inner surface of the valve body may each comprise one or more corresponding detent features configured to engage with one another to define the detent.

The outer surface of the valve core and the inner surface of the valve body may each comprise an undulating surface. The undulating surfaces may provide a pair of mating surfaces.

Each undulating surface may comprise a repeating substantially sinusoidal or triangular profile extending around the valve axis.

Each undulating surface may comprise a succession of peaks and troughs, or hills and valleys, extending around the valve axis. An apex of each peak, and a base of each trough, may extend laterally, or radially, from the valve axis.

At least one of the undulating surfaces may be formed as a plurality of concentric rings around the valve axis

The outer surface of the valve core and the inner surface of the valve body may comprise two corresponding cam surface profiles.

The outer surface of the valve core and the inner surface of the valve body may each have 90-degree rotational symmetry about the valve axis.

The detent may comprise a first rotational range and a second rotational range between the first stable configuration and the second stable configuration. The detent may bias the valve core towards the first stable configuration when the valve core is within the first rotational range. The detent may bias the valve core towards the second stable configuration when the valve core is within the second rotational range.

The actuator may comprise a lever rotatable about the valve axis and coupled to the valve core.

The valve body may further comprise a cap enclosing a first end of the valve body, wherein the cap comprises first and second stops to block rotation of the lever and valve core beyond the respective first and second stable configurations.

The valve core may be displaceable or slidable along the valve axis relative to the valve body and the lever.

The valve may be particularly suited for use with for breathing assistance apparatus, breathing assistance equipment and/or patient ventilator systems, which may be used synonymously herein. The first port may be configured for connection to a patient airway maintaining device. The second and third ports may be each configured for connection to a ventilator breathing circuit or breathing assistance device. The first port may have a 22 mm tapered outer diameter connector surface. The second and third ports may each have a 22 mm tapered internal diameter connector surface.

The valve as described above may further include a ventilation apparatus couplable to at least one of the second and third ports. The valve as described above may further include an endotracheal tube couplable to the first port.

According to a second aspect of the present disclosure there is provided a method of configuring breathing assistance apparatus for a patient comprising:
providing a valve comprising: a valve body defining first, second and third ports; a valve core defining a ported chamber rotatable about a valve axis between: a first stable configuration in which the ported chamber provides fluid communication between the first port and the second port and isolates the third port; and a second stable configuration in which the ported chamber provides fluid communication between the first port and the third port and isolates the second port, wherein an outer surface of the valve core is configured to engage with an inner surface of the valve body to define a detent for resisting rotation of the valve core between the first stable configuration and the second stable configuration; and an actuator overcome the detent to rotate the valve core between the first and second stable configurations;
coupling a patient breathing tube to the first port;
coupling a first ventilation device to the second port;
while the valve is in the first stable configuration, coupling a second ventilation device to the third port;
switching the valve to the second stable configuration; and
disconnecting the first ventilation device from the second port.

Also disclosed is a valve comprising:
first, second and third ports;
a bistable valve mechanism having (i) a first stable configuration in which the first port is in fluid communication with the second port and not the third port and (ii) a second stable configuration in which the first port is in fluid communication with the third port and not the second port; and
an actuator configured to transition the bistable valve mechanism between the two stable configurations and prevent the valve mechanism from maintaining a stable intermediate position between the first and second stable configurations. Various aspects of the valve are further described herein.

Embodiments of the present invention will now be described by way of example and with reference to the accompanying drawings in which:
Figure 1 shows a schematic diagram of a three-port connector in use with patient breathing assistance apparatus;
Figure 2A shows a perspective view of a three-port bistable valve for use with patient breathing assistance apparatus;
Figure 2B shows a cross-sectional view of the three-port bistable valve of figure 2A;
Figure 2C shows a perspective cut-away view of the three-port bistable valve of figure 2A;
Figures 3A and 3B respectively show a perspective view and a cross-sectional view of the bistable valve of figure 2A in a first stable configuration and figure 3C and 3D respectively show a perspective view and a cross-sectional view of the bistable valve of figure 2A in a second stable configuration;
Figure 3E shows a cross-sectional view of the bistable valve of Figure 2A in transition between the first stable configuration and the second stable configuration;
Figure 4A is an exploded perspective view, from the side and above, of the bistable valve of figure 2A;
Figure 4B is an exploded perspective view, from the side and below, of the bistable valve of figure 2A;
Figure 5 is an exploded cross-sectional schematic view of the valve of figure 2A useful in illustrating features of the bistable mechanism;
Figures 6A and 6B are exploded perspective views, respectively from the side and above and from the side and below, of another three-port bistable valve for use with patient breathing assistance apparatus;
Figure 6C is a perspective view showing the inner surfaces of the housing of the valve of Figures 6A and 6C;
Figures 7A and 7B are exploded perspective views, respectively from the side and above and from the side and below, of a further three-port bistable valve for use with patient breathing assistance apparatus;
Figure 7C is a perspective view showing the inner surfaces of the housing of the valve of Figures 7A and 7C;
Figure 7D is a cross section of the exploded perspective view shown in Figure 7A;
Figure 7E is a cross section of the assembled valve shown in Figure 7D; and
Figures 7F and 7G are perspective views of a core of the valve of Figures 7A to 7D.

Throughout the present specification, the descriptors relating to relative orientation and position, such as "top", "bottom", "horizontal", "vertical", "left", "right", "up", "down", "front", "back", as well as any adjective and adverb derivatives thereof, are used in the sense of the orientation of a valve as presented in the drawings. However, such descriptors are not intended to be in any way limiting to an intended use of the described or claimed invention. The expression 'clockwise' and 'anticlockwise' are used herein in the sense of directionality as viewed from above the valve, for the purpose of illustration and explanation of specific embodiments as illustrated, and are not intended to limit to those specific embodiments.

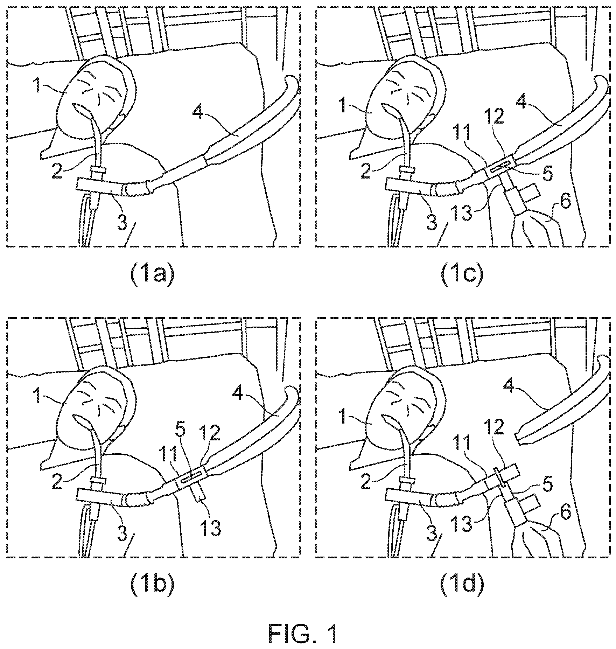

Figure 1 illustrates practical use of a patient ventilator system. As seen in figure 1a, patient 1 is provided with a breathing tube such as an endotracheal tube 2 placed through the mouth into the trachea to deliver oxygen and/or other gases to the lungs. Although the invention is described herein in connection with an endotracheal tube 2, other forms of breathing tube such as a tracheostomy tube can be applicable to the invention. A closed suction device 3 connects the patient to a ventilator breathing circuit 4. In circumstances as discussed above, it can be desirable to facilitate connection of the patient 1 to an alternative ventilation device, such as ventilation bag 6 (figures 1c and 1d). This can be achieved using a T-shaped valve 5 as seen in figure 1b which has first branch 11 connected to the patient breathing tube 2 (via the suction device 3), a second branch 12 connected to the ventilator breathing circuit 4 and a third branch 13 suitable for connecting to a further ventilation device. In figure 1b, the T-shaped valve 5 has a valve mechanism rotated to a first configuration in which the first branch 11 is fluidly coupled to the second branch 12 by way of a first fluid passage and in which the third branch 13 is isolated from the first fluid passage between the first branch 11 and the second branch 12.

As seen in figure 1c, an alternative ventilation device 6 is coupled to the third branch 13 while the valve mechanism remains in the first configuration coupling the first branch 11 and the second branch 12.

As seen in figure 1d, the valve mechanism is then rotated to a second configuration in which the first branch 11 is fluidly coupled to the third branch 13 by way of a second fluid passage and in which the second branch 12 is isolated from the second fluid passage between the first branch and 11 and the third branch 13. In this configuration, the ventilator breathing circuit 4 is then disconnected as seen in figure 1d. The disconnection occurs after the patient's airway has been connected to the third branch 13 of the T-shaped valve and thus to the alternative ventilation device 6 and isolated from the ventilator breathing circuit 4.

In one aspect, a valve suitable for implementation in the context of figures 1a - 1d may have a mechanism in which the transition from the first configuration to the second configuration occurs quickly such that the valve does not remain, for any appreciable period of time, in an intermediate position between the first configuration and the second configuration. The expression "intermediate position" may encompass a position in which fluid communication between the first branch 11 and the second branch 12 and the third branch 13 might coexist simultaneously, or a position in which there is no fluid communication between any of the first branch 11, the second branch 12 and the third branch 13 (the fluid pathway between each of the first, second and third branches is blocked). The expression "any appreciable length of time" may be understood to be relative to the normal pressure changes within a patient's airway. In another aspect, a valve suitable for implementation in the context of figures 1a to 1d may have a mechanism in which a user cannot statically position or leave the valve in such an intermediate position.

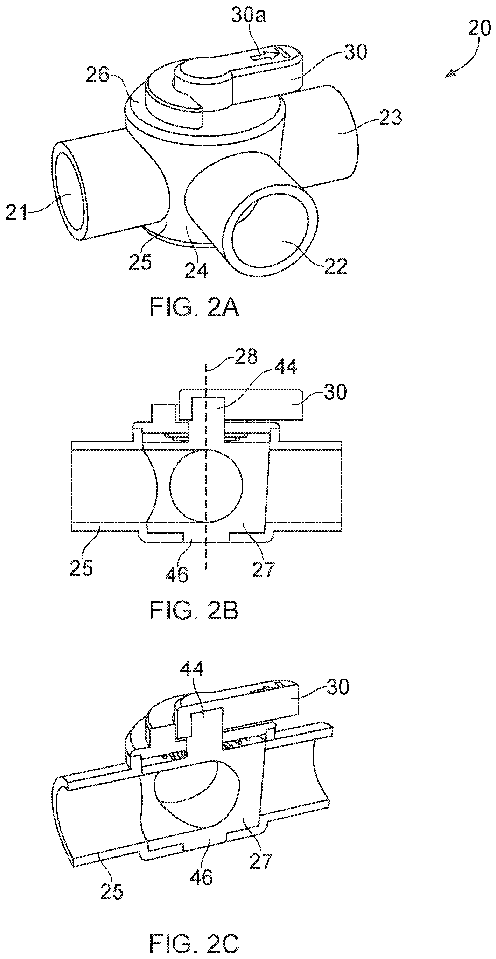

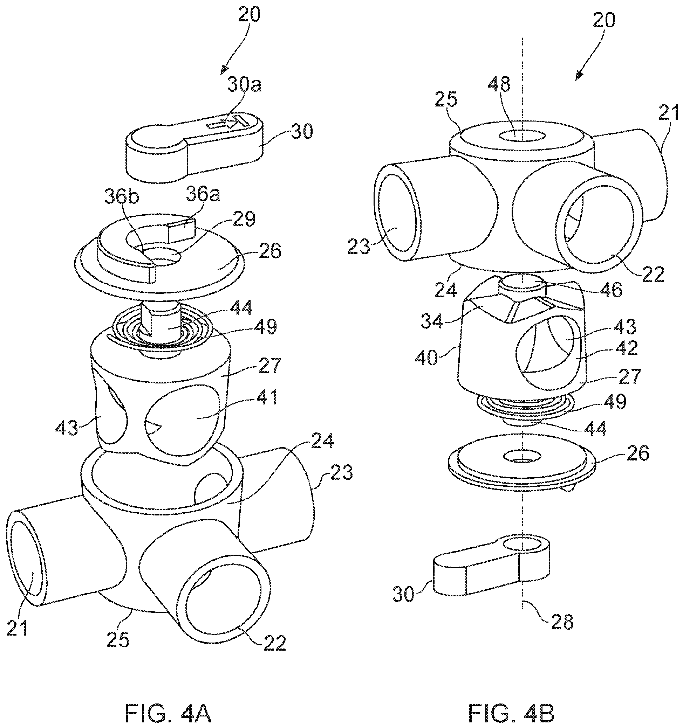

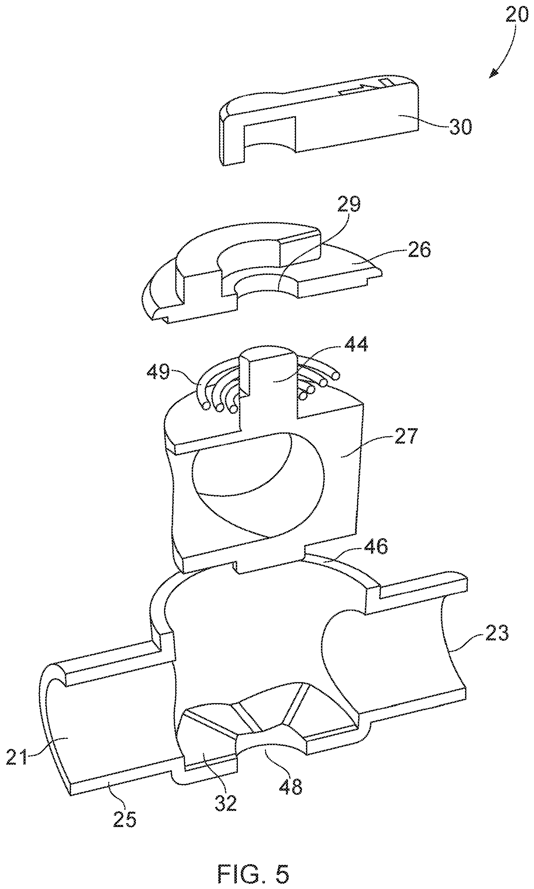

With reference to figures 2A - 5, a first example valve 20 suitable for the above purposes is described. Turning to figure 4A, the valve 20 has a first end (at the top of the figure) and a second end (at the bottom of the figure). The valve comprises an operating level 30, a valve cap 26, a valve core 27 and a valve body 25. The operating level 30 is provided at the first end and the valve body 25 is provided at the second end, in this example.

The valve body 25 defines three ports 21, 22, 23 extending radially outward from a cylindrical core portion 24 of the valve body 25. In this example, the valve cap 26 and body 25 encloses valve core 27 within the core portion 24 of the body 25. The cap 26 may be sealed to the valve body 25 via welding or other suitable processes known in the art.

The valve core 27 is rotatably mounted within the core portion 24 and rotatable about a valve axis 28 that extended between the first and second ends of the valve 20. As best seen with reference to figures 4A and 4B, the valve core 27 defines a ported chamber 40 having openings 41, 42, 43 respectively at 0, 180 and 90 degree positions relative to one another about the valve axis 28. These openings 41, 42, 43 are positioned to generally align with selected ones of the ports 21, 22, 23 when in certain angular positions within the valve body 25, as will be discussed later. For example, in a first stable configuration, the ported chamber 40 can provide fluid communication between a first port 21 and a second port 22 via a second opening 43 and a first opening 41. In a second stable configuration, the ported chamber 40 can provide fluid communication between the first port 21 and a third port 23 via the first opening 41 and a third opening 42.

Also seen in figure 4a is a central shaft of the valve core 27 that extends along the valve axis 28. The central shaft is discontinuous in this example. A first portion of the central shaft 44 is provided on a first surface of the valve core 27 (facing the first end of the valve 20) and a second portion of the central portion 34 is provided on an opposing second surface of the valve core 27 (facing the second end of the valve).

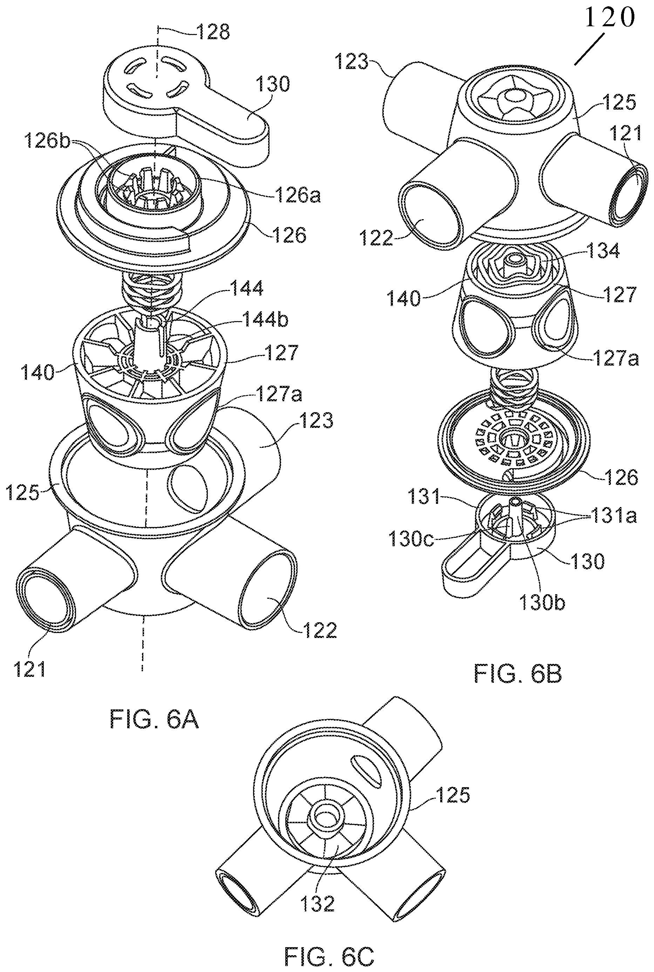

The first portion of the central shaft 44 extends axially through an aperture 29 in the cap 26 to engage with the operating lever 30 when the valve core 27 is installed within the body 25. The operating lever 30 is rotatable about the valve axis 28 and extends radially outwards from the cap 26. The first portion of a shaft 44 can slidably rotate within the aperture 29 such that the operating lever 30 is rotatable about the valve axis 28 relative to the cap 26 and valve body 25. In the arrangement shown, motion of the operating lever 30 is limited to an arc of approximately 90 degrees by stops 36a, 36b formed on an outer surface of the cap 26. The operating lever 30 is rotatably coupled to the valve core 27 such that rotation of the operating lever 30 also rotates the valve core 27. In this example, the operating lever 30 has a groove 45 for receiving the first portion of the shaft 44, as best seen in Figures 2B and 2C. The first portion of the shaft 44 has a chamfered edge 44a and the groove 45 has a corresponding profile to provide the rotatable engagement between the operating lever 30 and the valve core 27. In other examples (see Fig. 6, for example) the valve core 27 may comprise a groove or slot instead of a shaft and the operating lever 30 may include an engaging shaft for coupling to the groove, to provide the rotatable coupling. The operating lever 30 may include a direction indicator 30a to indicate the current configuration of the valve 20. In this example, the direction indicator 30a comprises an arrow pointing towards the port that is not in fluid communication with the first port.

Although the valve core 27 is rotatable coupled to the operating lever 30, the valve core 27 is slidably coupled to the operating lever 30 (and the valve body 25 and cap 26) in the axial direction such that the valve core 27 can be slidably displaced along the valve axis 28 relative to the valve body 25, the cap 26 and the operating lever 30.

An outer surface of the valve core 27 is provided facing an inner surface of the valve body 25 at the second end of the valve. The outer surface of the valve core 27 comprises detent features in the form of an undulating surface 34 (Figure 4B). In this example, the undulating surface 34 comprises a repeating triangular profile (or ramp profile) extending around the valve axis 28. In other examples, the undulating surface 34 may comprise a substantially sinusoidal profile extending around the valve axis. More generally, each undulating surface may comprise a succession of peaks and troughs, or hills and valleys, extending around the valve axis. An apex of each peak, and a base of each trough, may extend radially from the valve axis.

The inner surface of the valve body 25 comprises a corresponding undulating surface 32 such that the undulating surfaces 32, 34 can be mated together. The outer surface 34 of the valve core 27 is therefore configured to engage with the inner surface 32 of the valve body 25 to define a detent. The detent resists rotation of the valve core 27 between the first stable configuration and the second stable configuration. Both undulating surfaces 32, 34 have a 90 degree rotational symmetry about the valve axis 28. As a result, the two undulating surfaces 32, 34 comprise four positions in which they fully engage or mate. Two of these positions define the first and second stable configurations. In other words, the outer surface 34 of the valve core 27 is fully engaged with the inner surface 32 of the valve body 25 when the valve 20 is in the first stable configuration and the second stable configuration. In this example, fully engaged corresponds to the peaks of the triangular profile of the outer surface 34 of the valve core 32 nestling in the troughs of the triangular profile of the inner surface 32 of the valve body 25 and vice versa. In some examples, a resistive strength of the detent may be adjusted by selecting a ramp angle / amplitude of the undulating profiles 32, 34.

In this example, a second portion of the central shaft 46 extends from the outer surface of the valve core 27. The second portion of the central shaft 46 engages with a corresponding recess or aperture 48 in the surface of the valve body 25. The second portion of the central shaft 46 can slidably rotate within the aperture 48 as the valve core 27 rotates within the valve body 25 about the valve axis 28. The first and second portions of the shaft 44, 46 can provide stable rotation of the valve core 27 within the valve body and can prevent radial displacement of the valve core 27. It will be appreciated that in other examples, stable rotation of the valve core 27 within the valve body 25 about the valve axis 28 may be provided in other ways, for example with a single shaft extending through the valve core 27 or by central grooves in the first and / or second surfaces of the valve core 27 that engage with corresponding shafts in the operating lever 30 and / or inner surface of the valve body 25 as appropriate. However, all arrangements may provide slidable axial coupling permitting axial displacement of the valve core 27 relative to the valve body 25, the cap 26 and the operating lever 30.

In this example, the valve 20 further comprises a biasing element 49, in the form of a spring, positioned between the cap 26 and the valve core 27. The spring may be provided by a metal, rubber or plastic material. When the valve is assembled, the biasing element 49 exerts a biasing force on the valve core 27 to facilitate engagement of the outer surface 34 of the valve core 27 and the inner surface 32 of the valve body 25. In other words, the biasing element 49 pushes the valve core towards the valve body 25. As a result, the biasing element 49 can increase the strength of the detent in resisting rotation of the valve core 27 between the two stable configurations. A biasing force of the biasing element 49 may be selected based on a desired detent resistance.

In this example, the valve core 27 / ported chamber is tapered (as best seen in Fig. 2B) such that the second end of the valve core 27 is narrower than the first end of the valve core 27. In this example, the cylindrical portion 24 of the valve body 25 is also tapered. As such, the valve core 27 is substantially frustoconical. As will be discussed below, the tapered profile of the valve core 27 and valve body 25 can facilitate sealing between the openings 41, 42, 43 and the ports 21, 22, 23 when the valve is in the first and second stable configurations and reduce friction on the sealing faces to facilitate rotation when the valve core is positioned between the first and second configurations.

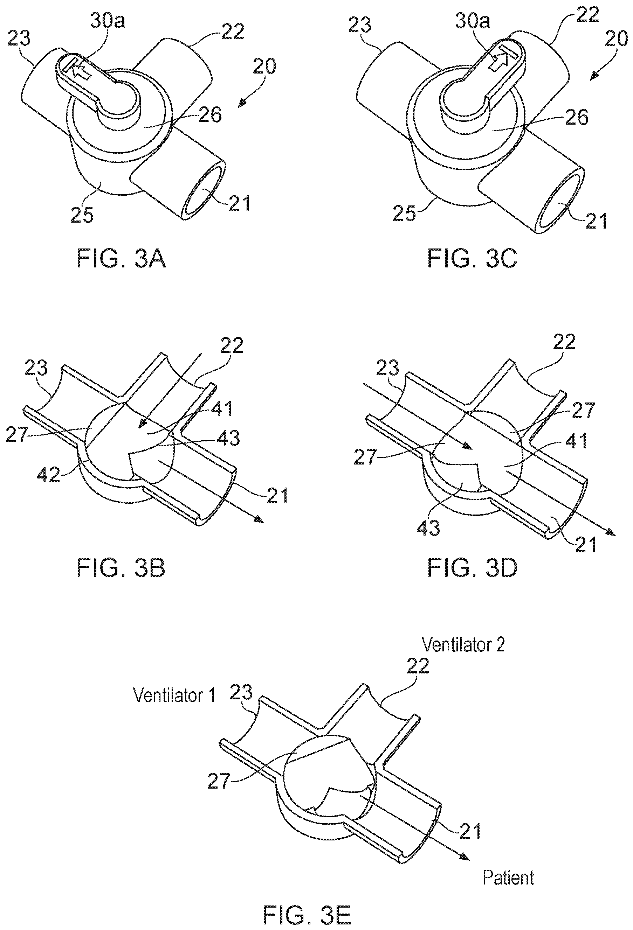

Figures 3A and 3B show the valve 20 in the first configuration and figures 3C and 3D show the valve 20 in the second configuration. In figures 3A and 3B, the valve 20 is in a first stable configuration in which the first port 21 is in fluid communication with the second port 22 via the ported chamber 40 of the valve core 27, because second opening 43 is in alignment with the first port 21 and first opening 41 is in alignment with second port 22. Third opening 42 is occluded by a wall of the valve body 25. It can also be seen that the third port 23 is blocked or occluded by the valve core 27 and therefore not in fluid communication with either the first port or the second port.

In figures 3C and 3D, the valve 20 is in a second stable configuration in which the first port 21 is in fluid communication with the third port 23 via the ported chamber 40 of the valve core 27, because first opening 41 is in alignment with the first port 21 and the third opening 42 is in alignment with the third port 23. The second opening 43 is occluded by a wall of the valve body 25. It can also be seen that the second port 22 is blocked or occluded by the valve core 27 and therefore not in fluid communication with either the first port or the third port. It can be understood from this diagram that in performing the quarter-turn anticlockwise rotation of the valve core 27 when transitioning from the configuration of figures 3A/3B to the configuration of figures 3C/3D, dependent on the diameters of the openings 41-43 and the ports 21-23, and on the circumference of the valve core 27 / cylindrical core portion 24, the valve 20 can be configured as either a "break-before-make" type valve where the fluid flow from one port pair is completely shut off before the other fluid pair communication starts to be made, or vice versa ("make-before-break").

Now also referring to figure 3E, the operation of the valve will be described.

Initially, the valve 20 is in the first stable configuration of Figures 3A and 3B with the operating lever 30 aligned with the third port 23 to indicate that the third port 23 is isolated and the first port 21 is in fluid communication to with the second port 22. The undulating outer surface 34 of the valve core 27 is fully engaged with the corresponding undulating inner surface 32 of the valve body 25. As a result, the valve core is at its closest to the inner surface 32 within the valve body 25. The tapered profile of the valve core 27 fits snuggly in the tapered profile of the valve body and can provide hermetic sealing between the first port 21 and the second opening 43 and the second port 22 and the first opening 41. The biasing element 49 provides a biasing force to maintain the valve core 27 in this first stable configuration in the absence of any force applied to the lever 30.

From the position of figures 3A and 3B, the operating lever 30 is moved in a clockwise direction. Force is applied to the operating lever 30 (rotatably coupled to the valve core 27) to overcome the biasing force of the biasing element 49 and the detent formed by the engagement of the undulating surface 34 of the valve core 27 and the undulating surface 32 of the valve body 25. The valve core 27 rotates anticlockwise about the valve axis 28 within the valve body 25. As the valve core 27 rotates, the undulating surfaces 32, 34 move out of full engagement causing the valve core 27 to axially displace along the valve axis 28 towards the cap 26 and against the biasing force of the biasing element 49. The peaks of the triangular profile of the undulating surface 34 of the valve core 27 move away from the troughs and towards the peaks of the undulating surface 32 of the valve body 25. As the valve core 27 lifts (axially displaces) towards the cap 26, the valve core 27 separates from an inner sidewall surface of the cylindrical portion 24 due to the tapered profiles. As a result, the seal between the openings 41, 42, 43 and ports 21, 22, 23 is broken. The removal of the sealing and "snug fit" of the valve core 27 in the valve body 25 facilitates rotation of the valve core 27 due to a reduction in friction between the valve core 27 and the valve body 25.

During a first rotation range of the valve core 27 as the peaks of the two triangular surface profiles 32, 34 approach each other, the detent and the biasing element bias the valve core 27 towards the first stable configuration. In other words, if the rotational force is removed from the operating lever 30, the valve core will rapidly rotate back towards the first stable configuration. During a second rotation range of the valve core 27 as the peaks of the two triangular surface profiles pass each other, the detent and the biasing element bias the valve core 27 towards the second stable configuration. Therefore, once the valve core has been rotated beyond the first rotation range (and the valve core 27 has reached its uppermost axial position) and the detent has been overcome (the triangular peaks pass), the valve core 27 will rapidly rotate towards the second stable configuration due to both the biasing force and the detent.

As the valve core 27 arrives in the second stable configuration (Figures 3C and 3D) the valve core 27 displaces axially away from the cap 26 and the undulating surface 34 of the valve core 27 fully re-engages with the undulating surface 32 of the valve body 25, as facilitated by the biasing force. A snug fit is reformed between the valve core 27 and the valve body 25. The first opening 41 may hermetically seal with the first port 41 and the third opening 42 may hermetically seal with the third port 23. The operating lever 30 is aligned with the second port 22 to indicate that the second port 22 is isolated and the first port 21 is in fluid communication with the third port 23. Further rotation of the operating lever 30 is prevented by the stop 36b on the cap 26.

A corresponding reverse movement of the operating lever 30 through approximately 90 degrees clockwise from the position of figures 3C and 3D provides a corresponding reverse rotational movement of the valve core 27 from the second stable configuration of figures 3C, 3D back to the first stable configuration of figures 3A, 3B.

Thus, the valve 20 exemplifies a bistable valve mechanism comprising a valve core 27 defining a ported chamber 40 rotatable within a valve body 25 about a valve axis 28 between: (i) a first stable configuration in which the ported chamber 40 provides fluid communication between a first port 21 and a second port 22 of the valve body and isolates a third port 23 and (ii) a second stable configuration in which ported chamber 40 provides fluid communication between the first port 21 and a third port 23 of the valve body 25 and isolates the second port 22, wherein an outer surface 34 of the valve core 27 is configured to engage with an inner surface 32 of the valve body 25 to define a detent for resisting rotation of the valve core 27 between the first stable configuration and the second stable configuration. Furthermore, the operating lever 30 and associated features exemplifies an actuator which is configured to overcome the detent to rotate the valve core between the first and second stable configurations. The expression 'fluid communication' is intended to encompass arrangements in which air or other gas flow between the communicating ports 21, 22, or 23 via the valve core 27 is sufficient in volume and sufficiently unimpeded to have little or no impact on the operation of a breathing circuit delivering gas to a patient's lungs via a breathing tube.

Various modifications and adaptations may be made to the valve 20 as described above. Figures 6A to 6C illustrates a second example valve 120 suitable for the purposes described in relation to Figure 1. The valve 120 comprises substantially the same arrangement and operation as the five-piece valve of Figures 2 to 5. The above description of valve 20 applies equally to valve 120 with the exception of the specific features described here in relation to Figures 6A to 6C.

In this example, the valve core 127 further comprises a sealing portion 127a for improving the sealing between the ports 121, 122, 123 and the ported chamber 140 when the valve core 127 is in the first and second stable configuration. During rotation of the valve core 127 between the two stable configurations, as the valve core 127 axially displaces towards the cap 126, the valve core 127 and sealing portion 127a separate from the inner sidewall of the valve body to reduce friction and facilitate rapid rotation towards the first or second stable configuration. The sealing portion may comprise a flexible or compressible material such as a rubber or an elastomer.

In this example, the detent is also provided by an undulating outer surface 134 of the valve core 127 that engages with an undulating inner surface 132 of the valve body 125. In this example, the two undulating surfaces 132, 134 comprise a repeating sinusoidal ramp profile that extends around the valve axis 128. In this example, the undulating outer surface 134 of the valve core 127 is formed as a plurality of concentric rings each having an aligned sinusoidal ramp profile around the valve axis. Using a concentric profile as illustrated can reduce the total material required for manufacturing reducing costs and waste.

The valve 120 of Figures 6A to 6C also differs from the valve of Figures 2 to 5 in the coupling arrangement between the operating lever 130, the cap 126 and the valve core 127. However, the fundamental principle remains the same that the operating lever 130 is rotatably engaged with the valve core 127 such that both components rotate together about the valve axis 128. The operating lever 130 and valve core 130 are slidably coupled with the cap 126 such that both components can rotate relative to the cap 126. The coupling arrangement also permits axial displacement of the valve core 127 along the valve axis 128 relative to the valve body 125, cap 126 and operating lever 130.

The operating lever 130 comprises a sidewall 131 and a plurality of first clip portions 131a protruding from a surface of the operating lever 130. The cap 126 comprises a corresponding receiving wall 126a and a corresponding plurality of second clip portions 126b protruding from the cap 126. The sidewall 131 of the operating lever 130 can fit over the receiving wall 126a of the cap 126. The first clip portions 131a can slidably engage with the second clip portions 126b to secure the operating lever to the cap 126 while permitting relative rotation about the valve axis 128 and preventing relative radial displacement.

The operating lever 130 further comprises a central shaft 130b. The shaft 130b comprises two side flanges 130c extending radially from the shaft 130b to define a key profile. The valve core 127 comprises a corresponding central 144 with a slot 144b for engaging with the central shaft 130b of the operating lever. The key profile of the shaft 130b can rotatably engage with the slot 144b of the central shaft 144 of the valve core to provide rotatable coupling between the operating lever 130 and the valve core 127.

Both valves 20, 120 are exemplified as 5 piece components, however in other examples, the valves 20, 120 may comprise more or fewer components. For example, the valve core may comprise a first sub-component defining the detent surface 34, 134 and a second component defining the ported chamber.

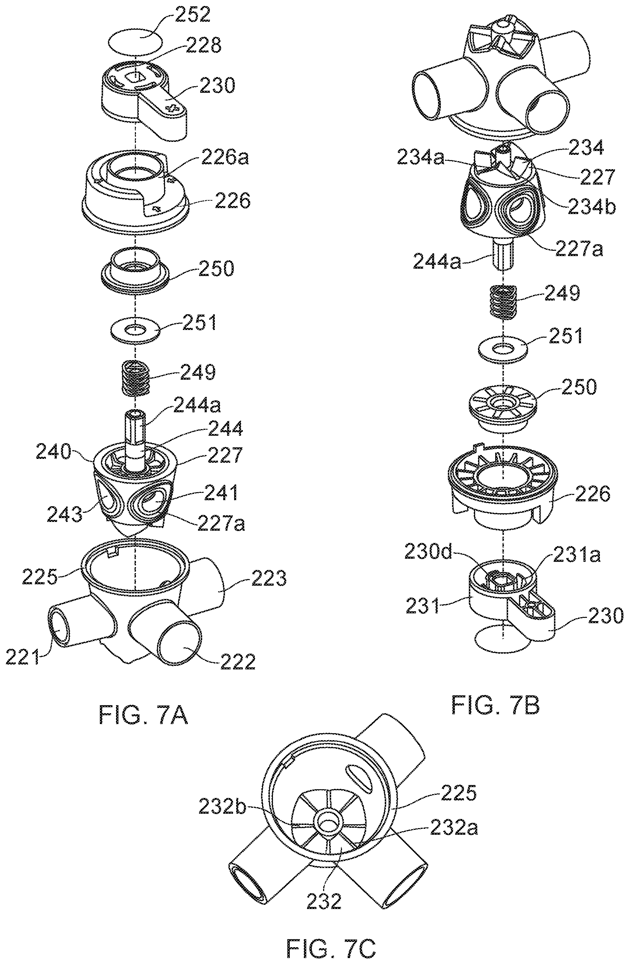

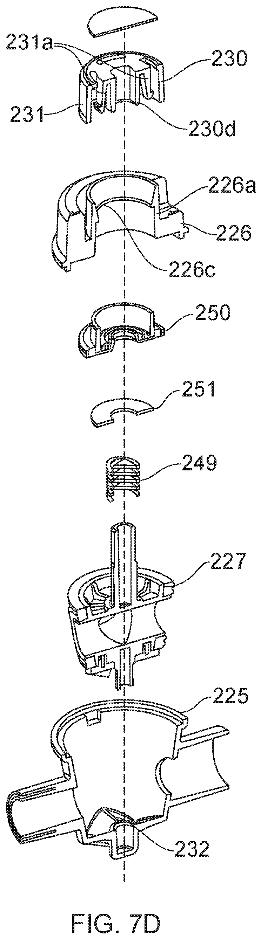

Figures 7A to 7G illustrate a third example valve 220 suitable for the purposes described in relation to Figure 1. The valve 220 comprises substantially the same arrangement and operation as the five-piece valve of Figures 2 to 5. The above description of valve 20 applies equally to valve 220 with the exception of the specific features described here in relation to Figures 7A to 7G.

In this example, the valve core 227 further comprises a sealing portion 227a for improving the sealing between the ports 221, 222, 223 and the ported chamber 240 when the valve core 227 is in the first and second stable configuration (in the same way as the valve of Figures 6A to 6C). During rotation of the valve core 227 between the two stable configurations, as the valve core 227 axially displaces towards the cap 226, the valve core 227 and sealing portion 227a separate from the inner sidewall of the valve body 225 to reduce friction and facilitate rapid rotation towards the first or second stable configuration. The sealing portion 227a may comprise a flexible or compressible material such as a rubber or an elastomer. In this example, a flexible material covers the entire conical surface of the ported chamber 240 and the sealing portions 227a comprise ridges in the flexible material that extend around the openings 241, 242, 243 in the ported chamber 240.

In this example, the detent is also provided by an undulating outer surface 234 of the valve core 227 that engages with an undulating inner surface 232 of the valve body 225. In this example, the two undulating surfaces 232, 234 comprise a repeating triangular ramp profile that extends around the valve axis 228. In this example, the undulating outer surface 234 of the valve core 227 includes flattened peaks 234a and sunken or etched valleys 234b. Similarly, the undulating surface 232 of the valve body 225 includes flattened peaks 232a and etched valleys 232b that engage with respective etched valleys 234b and flattened peaks 234a on undulating outer surface 234. These additional features can increase the strength of the detent and / or the stability of the first and second stable configurations.

The valve 220 of Figures 7A to 7G also differs from the valve of Figures 2 to 5 by comprising a cap sealing portion 250. The cap sealing portion 250 provides additional sealing to the cap 226 to prevent air leaks at the interface between the cap and the central shaft 244. The cap sealing portion 250 also provides a seal between the cap 226 and the air space inside the valve body 225 above the valve core 227. In this example, the cap sealing portion 250 includes an aperture for receiving the central shaft 244 therethrough. The aperture is formed with a flexible material, such as rubber or an elastomer, to provide an air tight seal. A washer 251 is positioned between the cap sealing portion 250 and the biasing element 249 to provide an engagement interface between the cap sealing portion 250 and the biasing element 249.

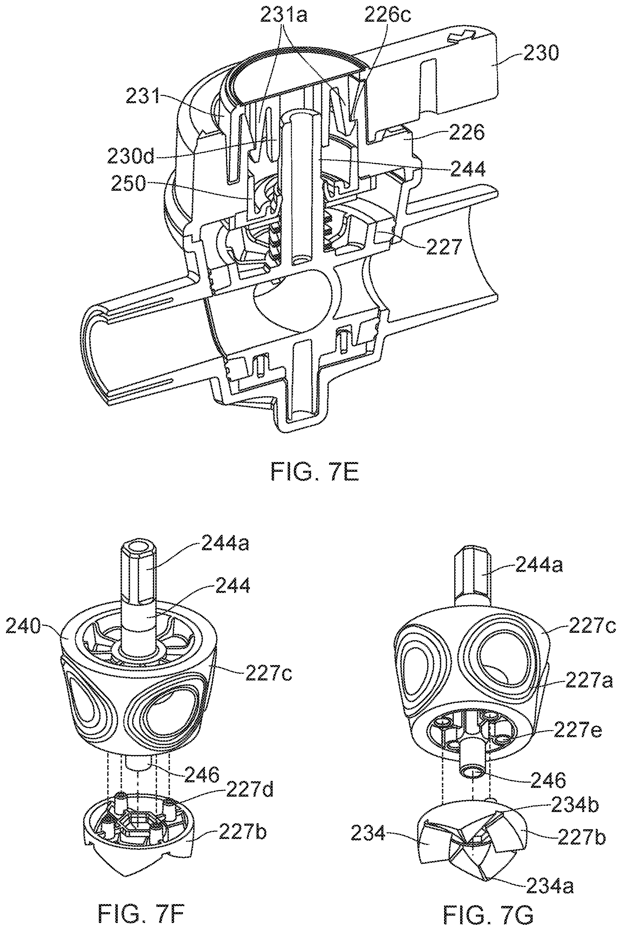

The valve 220 of Figures 7A to 7G also differs from the valve of Figures 2 to 5 by comprising a two part valve core 227 (best illustrated in Figures 7F and 7G). In this example, the valve core 227 comprises a first valve core component 227b defining the detent surface 234 and a second valve core component 227c defining the ported chamber 240 and central shaft 244, 246. The first valve core component 227b includes dowels 227d for engaging with corresponding dowel apertures 227e on the second valve core component 227c.

The valve 220 of Figures 7A to 7C also differs from the valve of Figures 2 to 5 in the coupling arrangement between the operating lever 230, the cap 226 and the valve core 227. However, the fundamental principle remains the same that the operating lever 230 is rotatably engaged with the valve core 227 such that both components rotate together about the valve axis 228. The operating lever 230 and valve core 227 are slidably coupled with the cap 226 such that both components can rotate relative to the cap 226. The coupling arrangement also permits axial displacement of the valve core 227 along the valve axis 228 relative to the valve body 225, cap 226 and operating lever 230. This is best illustrated in Figure 7E in which a height of a central channel 230d of the operating lever 230 is sufficiently large to permit axial displacement of the central shaft 244 within the central channel 230d as the valve core 227 rotates between the first and second stable configurations and displaces axially towards the cap 226 and then returns back again.

The operating lever 230 comprises a sidewall 231 and a plurality of first clip portions 231a protruding from a surface of the operating lever 230. The cap 226 comprises a corresponding receiving wall 226a. The sidewall 231 of the operating lever 230 can fit over the receiving wall 226a of the cap 226. The first clip portions 231a can slidably engage with an internal rim 226c of the receiving wall 226a to secure the operating lever 230 to the cap 226 while permitting relative rotation about the valve axis 228 and preventing relative radial displacement.

The central shaft 244 has chamfered edges 244a to define a substantially square cross-section. The central channel 230d of the operating lever includes a corresponding substantially square cross-sectional aperture for receiving the central shaft 244. The chamfered edges of the central shaft 144 can rotatably engage with the central channel 230d to provide rotatable coupling between the operating lever 230 and the valve core 227. The operating lever 230 also has a lever cap 252 for providing a cover for, and/or an end stop to, the internal central channel 230d.

The valves 20, 120, 220 described above can be configured to act as a patient breathing circuit connector and may be configured for particular use with standardised patent assisted breathing apparatus. This may include providing standard fittings on each of the first, second and third ports 21/121/221, 22/122/222, 23/123/223. For example, the first port 21, 121, 221 may be configured for connection to a patient airway maintaining device / breathing tube such as an endotracheal tube 2 or a tracheostomy tube, or via a suction device 3 connected thereto. In this respect, the first port 21, 121, 221 may preferably comprise a male connector with a conical 22 mm outer diameter connector surface with a 1:40 taper and an internal co-axial conical connector with a 15 mm internal diameter (female) conical connector with a 1:40 taper.

The second port 22, 122, 222 may be configured for connection to a ventilator 4 via breathing circuit tubing and can be left open to air when not in use. The second port 22, 122, 222 may comprise a female connector with a conical 22 mm internal diameter connector surface with a 1:40 taper. The third port 23, 123, 223 may be configured for connection to a second breathing circuit 6 and can also be left open to air when not in use. The third port 23, 123, 223 may comprise a female connector with a conical 22 mm internal diameter connector surface with a 1:40 taper. In this respect, the valve may be specifically configured to be compatible with relevant national and/or international standards, such as ISO 5356-1:2004 or ISO 5356-1:2015 specifying dimensional and gauging requirements for cones and sockets intended for connecting anaesthetic and respiratory equipment, e.g. in breathing systems, anaesthetic-gas scavenging systems and vaporizers.

The operating lever 30, 130, 230 allows switching from one breathing circuit 4 to another breathing circuit 6 while maintaining a closed circuit, thus preventing the release of potentially infected aerosols and minimizing the loss of positive end-expiratory pressure (PEEP) from the circuit. The valve may preferably be formed as a T-shaped device with ports at 90 / 180 degree relative angles, though other angular dispositions of the ports can be envisaged.

Valves 20, 120, 220 may be formed from a range of materials. In some examples, one or more components of the valve 20, 120, 220 may be formed from the same materials. The valve is preferably made principally from strong, lightweight plastic, e.g. with injection moulded parts, and is preferably a disposable device used for a single patient and for a limited period of time, e.g. up to a few days. Example materials may include polymers such as polyethylene, polysulfone, polyacetal and polystyrene butadiene.

The valve preferably transitions from one bistable state to the other bistable state in a period of time which is very small compared to the period of normal pressure changes within a patient's airway, e.g. substantially less than 1 second and preferably substantially less than 0.2 second.

The valve arrangements as described herein provide fast-acting transitions with a decisive and unambiguous snap action which provides strong haptic and audible feedback when the valve transitions between its bistable states. This is particularly useful in the clinical context for ensuring ease of use in a potentially noisy and confusing environment. As a further safety feature, the direction indicator 30a also provides clear visual feedback as to the status of the valve and cannot show an ambiguous intermediate indication as it is integrally formed with the operating lever which is rotatably coupled to the valve core 27.

In the embodiments shown herein, a three-port valve configuration is shown exemplifying a bistable valve mechanism in which a first port can be connected to either one of a second and third port by a rapidly transitioning valve mechanism. It will be understood that further ports could be added, and the actuator mechanism supplemented with a third position e.g. to provide a tristable valve mechanism. Such a tristable valve could have a position in which the first port can be connected to any one of a second, third or fourth port, or there could be a third stable position corresponding to a full isolated position where the first, second and third ports are all isolated from one another. In this respect, the expression 'bistable valve' is intended to encompass a valve having at least a first and a second stable configuration in which an intermediate stable position between the first and second stable configurations is prevented, but need not exclude the possibility of having a further stable configuration while still meeting the specified bistable requirements.

Although the embodiments of valve described in connection with the drawings are based on a bistable valve mechanism and actuator mechanism which rotate about an axis, it will be understood that the bistable functionality could also be achieved in other ways, e.g. with a valve mechanism deploying linear motion. For example, the valve could be configured as a Y-shaped valve with the stem of the Y-shape corresponding to the first port and the two branches of the Y-shape corresponding to the second and third ports. A linear sliding bistable mechanism may be used to transition the valve from a first configuration in which the first port is in fluid communication with the second port to a second configuration in which the first port is in fluid communication with the third port.

For the purposes of providing low cost disposable valves, the valves herein are preferably manually operated valves and constructed from low cost components. However, other electrically actuated versions may be envisaged.

The valve as described above can be deployed to more safely switch a patient using a breathing support tube and connected to a first ventilation device to a second ventilation device with reduced risk of the problems identified above. The patient's breathing tube is coupled to the first port of the valve and the first ventilation device is coupled to the second port and the breathing apparatus is operated with the valve in the first stable configuration. While the valve is in the first stable configuration, a second ventilation device may be coupled to the third port. The valve may then be switched to the second stable configuration. After the transition of the valve to the second stable configuration the first ventilation device may be disconnected from the second port which has been isolated from the patient's active breathing circuit.

Other embodiments are intentionally within the scope of the accompanying claims.

## Claims

1. A breathing assistance connector valve (20) comprising:
a valve body (25) defining first, second and third ports (21, 22, 23);
a valve core (27) defining a ported chamber (40) rotatable about a valve axis (28) between:
a first stable configuration in which the ported chamber (40) provides fluid communication between the first port (21) and the second port (22) and isolates the third port (23) from the first and second ports (21, 22); and
a second stable configuration in which the ported chamber (40) provides fluid communication between the first port (21) and the third port (23) and isolates the second port (22) from the first and third ports (21, 23),
wherein an outer surface (34) of the valve core (27) is configured to engage with an inner surface (32) of the valve body (25) to define a detent for resisting rotation of the valve core (27) between the first stable configuration and the second stable configuration; and
an actuator configured to overcome the detent to rotate the valve core (27) between the first and second stable configurations.

2. The valve of claim 1, comprising a biasing element (49) configured to exert a biasing force along the valve axis (28) to urge together the outer surface (34) of the valve core (27) and the inner surface (32) of the valve body (25).

3. The valve of claim 1 or claim 2, wherein the actuator is configured to overcome the detent and the biasing force such that the valve core (27):
rotates about the valve axis (28) between the first stable configuration and the second stable configuration; and
is displaced along the valve axis (28) away from the inner surface (32) of the valve body (25).

4. The valve of claim 3, wherein:
the valve core (27) comprises a tapered portion defining the ported chamber (40), wherein a second end of the tapered portion is narrower than a first end of the tapered portion; and
the valve body (25) comprises a tapered portion for receiving the tapered portion of the valve core (27).

5. The valve of any preceding claim, wherein the valve core (27) comprises one or more sealing portions (127a) on an outer surface (34) of the valve core (27) configured to provide a seal between the valve core (27) and the first, second and third ports (21, 22, 23).

6. The valve of any preceding claim, wherein the outer surface (34) of the valve core (27) and the inner surface (32) of the valve body (25) each comprise one or more corresponding detent features configured to engage with one another to define the detent,
optionally wherein the outer surface (34) of the valve core (27) and the inner surface (32) of the valve body (25) each comprise an undulating surface,
wherein each undulating surface preferably comprises a repeating substantially sinusoidal or triangular profile extending around the valve axis (28), and
wherein at least one of the undulating surfaces is further preferably formed as a plurality of concentric rings around the valve axis (28).

7. The valve of any preceding claim, wherein the outer surface (34) of the valve core (27) and the inner surface (32) of the valve body (25) each have 90-degree rotational symmetry about the valve axis (28).

8. The valve of any preceding claim, wherein the detent comprises a first rotational range and a second rotational range between the first stable configuration and the second stable configuration and wherein:
the detent biases the valve core (27) towards the first stable configuration when the valve core (27) is within the first rotational range; and
the detent biases the valve core (27) towards the second stable configuration when the valve core (27) is within the second rotational range.

9. The valve of any preceding claim, wherein the actuator comprises a lever (130) rotatable about the valve axis (28) and coupled to the valve core (27).

10. The valve of claim 9, wherein the valve body (25) further comprises a cap (26) enclosing a first end of the valve body, wherein the cap (26) comprises first and second stops to block rotation of the lever (130) and valve core (27) beyond the respective first and second stable configurations.

11. The valve of claim 9 or claim 10, wherein the valve core (27) is displaceable along the valve axis (28) relative to the valve body (25) and the lever (130).

12. The valve of any preceding claim wherein the first port (21) is configured for connection to a patient airway maintaining device (2, 3), and the second and third ports (22, 23) are each configured for connection to a ventilator breathing circuit (4) or breathing assistance device.

13. The valve of claim 12 in which the first port (21) has a 22 mm tapered outer diameter connector surface and the second and third ports (22, 23) each have a 22 mm tapered internal diameter connector surface.

14. A system comprising:
the valve (20) of any preceding claim;
a ventilation apparatus couplable to at least one of the second and third ports (22, 23); and/or
an endotracheal tube (2) couplable to the first port (21).

15. A method of configuring breathing assistance apparatus for a patient comprising:
providing a valve (20) comprising: a valve body (25) defining first, second and third ports (21, 22, 23); a valve core (27) defining a ported chamber (40) rotatable about a valve axis (28) between: a first stable configuration in which the ported chamber (40) provides fluid communication between the first port (21) and the second port (22) and isolates the third port (23); and a second stable configuration in which the ported chamber (40) provides fluid communication between the first port (21) and the third port (23) and isolates the second port (22), wherein an outer surface (34) of the valve core (27) is configured to engage with an inner surface (32) of the valve body (25) to define a detent for resisting rotation of the valve core (27) between the first stable configuration and the second stable configuration; and an actuator overcome the detent to rotate the valve core (27) between the first and second stable configurations;
coupling a patient breathing tube to the first port (21);
coupling a first ventilation device to the second port (22);
while the valve (20) is in the first stable configuration, coupling a second ventilation device to the third port (23);
switching the valve (20) to the second stable configuration; and
disconnecting the first ventilation device from the second port (22).

## Patentansprüche

1. Verbinderventil für Atemhilfe (20), umfassend:
einen Ventilkörper (25), der eine erste, zweite und dritte Öffnung (21, 22, 23) definiert;
einen Ventilkern (27), der eine mit Öffnungen versehene Kammer (40) definiert, die um eine Ventilachse (28) drehbar ist zwischen:
einer ersten stabilen Konfiguration, in der die mit Öffnungen versehene Kammer (40) eine Fluidkommunikation zwischen der ersten Öffnung (21) und der zweiten Öffnung (22) bereitstellt und die dritte Öffnung (23) von der ersten und zweiten Öffnung (21, 22) isoliert; und
einer zweiten stabilen Konfiguration, in der die mit Öffnungen versehene Kammer (40) eine Fluidkommunikation zwischen der ersten Öffnung (21) und der dritten Öffnung (23) bereitstellt und die zweiten Öffnung (22) von der ersten und dritten Öffnung (21, 23) isoliert,
wobei eine äußere Oberfläche (34) des Ventilkerns (27) konfiguriert ist, um in eine innere Oberfläche (32) des Ventilkörpers (25) einzugreifen, um eine Arretierung zu definieren, die einer Drehung des Ventilkerns (27) zwischen der ersten stabilen Konfiguration und der zweiten stabilen Konfiguration widersteht; und
einen Aktuator, der konfiguriert ist, um die Arretierung zu überwinden, um den Ventilkern (27) zwischen den ersten und zweiten stabilen Konfigurationen zu drehen.

2. Ventil nach Anspruch 1, umfassend ein Vorspannelement (49), das konfiguriert ist, um eine Vorspannkraft entlang der Ventilachse (28) auszuüben, um die äußere Oberfläche (34) des Ventilkerns (27) und die innere Oberfläche (32) des Ventilkörpers (25) zusammenzudrängen.

3. Ventil nach Anspruch 1 oder 2, wobei der Aktuator konfiguriert ist, um die Arretierung und die Vorspannkraft zu überwinden, sodass der Ventilkern (27):
sich um die Ventilachse (28) zwischen der ersten stabilen Konfiguration und der zweiten stabilen Konfiguration dreht; und
entlang der Ventilachse (28) von der inneren Oberfläche (32) des Ventilkörpers (25) weg verlagert wird.

4. Ventil nach Anspruch 3, wobei:
der Ventilkern (27) einen verjüngten Abschnitt umfasst, der die mit Öffnungen versehene Kammer (40) definiert, wobei ein zweites Ende des verjüngten Abschnitts schmaler ist als ein erstes Ende des verjüngten Abschnitts; und
der Ventilkörper (25) einen verjüngten Abschnitt umfasst, um den verjüngten Abschnitt des Ventilkerns (27) zu empfangen.

5. Ventil nach einem der vorstehenden Ansprüche, wobei der Ventilkern (27) einen oder mehrere Dichtungsabschnitte (127a) auf einer äußeren Oberfläche (34) des Ventilkerns (27) umfasst, die konfiguriert sind, um eine Dichtung zwischen dem Ventilkern (27) und der ersten, zweiten und dritten Öffnung (21, 22, 23) bereitzustellen.

6. Ventil nach einem der vorstehenden Ansprüche, wobei die äußere Oberfläche (34) des Ventilkerns (27) und die innere Oberfläche (32) des Ventilkörpers (25) jeweils ein oder mehrere entsprechende Arretierungsmerkmale umfassen, die konfiguriert sind, ineinander einzugreifen, um die Arretierung zu definieren,
optional wobei die äußere Oberfläche (34) des Ventilkerns (27) und die innere Oberfläche (32) des Ventilkörpers (25) jeweils eine wellenförmige Oberfläche umfassen,
wobei jede wellenförmige Oberfläche vorzugsweise ein sich wiederholendes, im Wesentlichen sinusförmiges oder dreieckiges Profil umfasst, das sich um die Ventilachse (28) herum erstreckt, und
wobei mindestens eine der wellenförmigen Oberflächen ferner vorzugsweise als eine Vielzahl von konzentrischen Ringen um die Ventilachse (28) herum ausgebildet ist.

7. Ventil nach einem der vorstehenden Ansprüche, wobei die äußere Oberfläche (34) des Ventilkerns (27) und die innere Oberfläche (32) des Ventilkörpers (25) jeweils eine 90-Grad-Rotationssymmetrie um die Ventilachse (28) herum aufweisen.

8. Ventil nach einem der vorstehenden Ansprüche, wobei die Arretierung einen ersten Drehbereich und einen zweiten Drehbereich zwischen der ersten stabilen Konfiguration und der zweiten stabilen Konfiguration umfasst und wobei:
die Arretierung den Ventilkern (27) in Richtung der ersten stabilen Konfiguration vorspannt, wenn sich der Ventilkern (27) innerhalb des ersten Drehbereichs befindet; und
die Arretierung den Ventilkern (27) in Richtung der zweiten stabilen Konfiguration vorspannt, wenn sich der Ventilkern (27) innerhalb des zweiten Drehbereichs befindet.

9. Ventil nach einem der vorstehenden Ansprüche, wobei der Aktuator einen Hebel (130) umfasst, der um die Ventilachse (28) herum drehbar und mit dem Ventilkern (27) gekoppelt ist.

10. Ventil nach Anspruch 9, wobei der Ventilkörper (25) ferner eine Kappe (26) umfasst, die ein erstes Ende des Ventilkörpers umschließt, wobei die Kappe (26) einen ersten und einen zweiten Anschlag umfasst, um die Drehung des Hebels (130) und des Ventilkerns (27) über die jeweiligen ersten und zweiten stabilen Konfigurationen hinaus zu blockieren.

11. Ventil nach Anspruch 9 oder 10, wobei der Ventileinsatz (27) entlang der Ventilachse (28) relativ zu dem Ventilkörper (25) und dem Hebel (130) verschiebbar ist.

12. Ventil nach einem der vorstehenden Ansprüche, wobei die erste Öffnung (21) für die Verbindung mit einer Vorrichtung zur Aufrechterhaltung der Atemwege eines Patienten (2, 3) konfiguriert ist und die zweite und dritte Öffnung (22, 23) jeweils für die Verbindung mit einem Beatmungsschlauchsystem (4) oder einer Atemunterstützungsvorrichtung konfiguriert sind.

13. Ventil nach Anspruch 12, bei dem die erste Öffnung (21) eine sich verjüngende Verbinderoberfläche mit einem Außendurchmesser von 22 mm aufweist und die zweite und dritte Öffnung (22, 23) jeweils eine sich verjüngende Verbinderoberfläche mit einem Innendurchmesser von 22 mm aufweisen.

14. System, umfassend:
das Ventil (20) nach einem der vorstehenden Ansprüche;
ein Belüftungsgerät, das mit mindestens einer der zweiten und dritten Öffnungen (22, 23) verbunden werden kann; und/oder
einen Endotrachealschlauch (2), der mit der ersten Öffnung (21) verbunden werden kann.

15. Verfahren zum Konfigurieren eines Atemhilfegeräts für Patienten, umfassend:
Bereitstellen eines Ventils (20), umfassend: einen Ventilkörper (25), der erste, zweite und dritte Öffnungen (21, 22, 23) definiert; einen Ventilkern (27), der eine mit Öffnungen versehene Kammer (40) definiert, die um eine Ventilachse (28) herum drehbar ist zwischen: einer ersten stabilen Konfiguration, in der die mit Öffnungen versehene Kammer (40) eine Fluidkommunikation zwischen der ersten Öffnung (21) und der zweiten Öffnung (22) bereitstellt und die dritte Öffnung (23) isoliert; und
einer zweiten stabilen Konfiguration, in der die mit Öffnungen versehene Kammer (40) eine Fluidkommunikation zwischen der ersten Öffnung (21) und der dritten Öffnung (23) bereitstellt und die zweite Öffnung (22) isoliert, wobei eine äußere Oberfläche (34) des Ventilkerns (27) konfiguriert ist, um in eine innere Oberfläche (32) des Ventilkörpers (25) einzugreifen, um eine Arretierung zu definieren, die einer Drehung des Ventilkerns (27) zwischen der ersten stabilen Konfiguration und der zweiten stabilen Konfiguration widersteht; und einen Aktuator, der die Arretierung überwindet, um den Ventilkern (27) zwischen den ersten und zweiten stabilen Konfigurationen zu drehen;
Koppeln eines Patientenatemschlauchs an die erste Öffnung (21);
Koppeln einer ersten Beatmungsvorrichtung mit der zweiten Öffnung (22);
während sich das Ventil (20) in der ersten stabilen Konfiguration befindet, Koppeln einer zweiten Beatmungsvorrichtung mit der dritten Öffnung (23);
Umschalten des Ventils (20) in die zweite stabile Konfiguration; und
Abtrennen der ersten Beatmungsvorrichtung von der zweiten Öffnung (22).

## Revendications

1. Vanne de raccordement pour assistance respiratoire (20) comprenant :
un corps de vanne (25) définissant les premier, deuxième et troisième orifices (21,22, 23) ;
un noyau de vanne (27) définissant une chambre à orifice (40) pouvant tourner autour d'un axe de vanne (28) entre :
une première configuration stable dans laquelle la chambre à orifice (40) fournit une communication fluidique entre le premier orifice (21) et le deuxième orifice (22) et isole le troisième orifice (23) des premier et deuxième orifices (21,22) ; et
une seconde configuration stable dans laquelle la chambre à orifice (40) fournit une communication fluidique entre le premier orifice (21) et le troisième orifice (23) et isole le deuxième orifice (22) des premier et troisième orifices (21,23),
dans laquelle une surface externe (34) du noyau de vanne (27) est conçue pour se mettre en prise avec une surface interne (32) du corps de vanne (25) afin de définir un encliquetage permettant de résister à la rotation du noyau de vanne (27) entre la première configuration stable et la seconde configuration stable ; et
un actionneur conçu pour surmonter l'encliquetage afin de faire tourner le noyau de vanne (27) entre les première et seconde configurations stables.

2. Vanne selon la revendication 1, comprenant un élément de sollicitation (49) conçu pour exercer une force de sollicitation le long de l'axe de vanne (28) afin de pousser ensemble la surface externe (34) du noyau de vanne (27) et la surface interne (32) du corps de vanne (25).

3. Vanne selon la revendication 1 ou la revendication 2, dans laquelle l'actionneur est conçu pour surmonter l'encliquetage et la force de sollicitation de telle sorte que le noyau de vanne (27) :
tourne autour de l'axe de vanne (28) entre la première configuration stable et la seconde configuration stable ; et
est déplacé le long de l'axe de vanne (28) à l'écart de la surface interne (32) du corps de vanne (25).

4. Vanne selon la revendication 3, dans laquelle :
le noyau de vanne (27) comprend une partie conique définissant la chambre à orifice (40), dans laquelle une deuxième extrémité de la partie conique est plus étroite qu'une première extrémité de la partie conique ; et
le corps de vanne (25) comprend une partie conique permettant de recevoir la partie conique du noyau de vanne (27).

5. Vanne selon l'une quelconque revendication précédente, dans laquelle le noyau de vanne (27) comprend une ou plusieurs parties d'étanchéité (127a) sur une surface externe (34) du noyau de vanne (27) conçues pour fournir une étanchéité entre le noyau de vanne (27) et les premier, deuxième et troisième orifices (21, 22, 23).

6. Vanne selon l'une quelconque revendication précédente, dans laquelle la surface externe (34) du noyau de vanne (27) et la surface interne (32) du corps de vanne (25) comprennent chacune un ou plusieurs éléments d'encliquetage correspondants conçus pour se mettre en prise les uns avec les autres afin de définir l'encliquetage,
éventuellement, dans laquelle la surface externe (34) du noyau de vanne (27) et la surface interne (32) du corps de vanne (25) comprennent chacune une surface ondulée, dans laquelle chaque surface ondulée comprend de préférence un profil répétitif sensiblement sinusoïdal ou triangulaire s'étendant autour de l'axe de vanne (28), et
dans laquelle au moins l'une parmi les surfaces ondulées est de préférence formée comme une pluralité d'anneaux concentriques autour de l'axe de vanne (28).

7. Vanne selon l'une quelconque revendication précédente, dans laquelle la surface externe (34) du noyau de vanne (27) et la surface interne (32) du corps de vanne (25) ont chacune une symétrie de rotation de 90 degrés autour de l'axe de vanne (28).

8. Vanne selon l'une quelconque revendication précédente, dans laquelle l'encliquetage comprend une première plage de rotation et une seconde plage de rotation entre la première configuration stable et la seconde configuration stable, et dans laquelle :
l'encliquetage sollicite le noyau de vanne (27) vers la première configuration stable lorsque le noyau de vanne (27) se trouve dans la première plage de rotation ; et
l'encliquetage sollicite le noyau de vanne (27) vers la seconde configuration stable lorsque le noyau de vanne (27) se trouve dans la seconde plage de rotation.

9. Vanne selon l'une quelconque revendication précédente, dans laquelle l'actionneur comprend un levier (130) pouvant tourner autour de l'axe de vanne (28) et accouplé au noyau de vanne (27).

10. Vanne selon la revendication 9, dans laquelle le corps de vanne (25) comprend en outre un capuchon (26) recouvrant une première extrémité du corps de vanne, dans laquelle le capuchon (26) comprend une première et une seconde butées pour bloquer la rotation du levier (130) et du noyau de vanne (27) au-delà des première et deuxième configurations stables respectives.

11. Vanne selon la revendication 9 ou la revendication 10, dans laquelle le noyau de vanne (27) peut être déplacé le long de l'axe de vanne (28) par rapport au corps de vanne (25) et au levier (130).

12. Vanne selon l'une quelconque revendication précédente dans laquelle le premier orifice (21) est conçu pour être raccordé à un dispositif de maintien des voies respiratoires d'un patient (2, 3), et les deuxième et troisième orifices (22, 23) sont chacun conçus pour être raccordés à un circuit respiratoire de ventilation (4) ou à un dispositif d'assistance respiratoire.

13. Vanne selon la revendication 12 dans laquelle le premier orifice (21) a une surface de raccordement à diamètre externe conique de 22 mm et les deuxième et troisième orifices (22, 23) ont chacun une surface de raccordement à diamètre interne conique de 22 mm.

14. Système comprenant :
la vanne (20) selon l'une quelconque revendication précédente ;
un appareil de ventilation pouvant être accouplé à au moins l'un parmi les deuxième et troisième orifices (22, 23) ; et/ou
un tube endotrachéal (2) pouvant être accouplé au premier orifice (21).

15. Procédé de configuration d'un appareil d'assistance respiratoire pour un patient, comprenant : la fourniture d'une vanne (20) comprenant : un corps de vanne (25) définissant les premier, deuxième et troisième orifices (21, 22, 23) ; un noyau de vanne (27) définissant une chambre à orifice (40) pouvant tourner autour d'un axe de vanne (28) entre : une première configuration stable dans laquelle la chambre à orifice (40) fournit une communication fluidique entre le premier orifice (21) et le deuxième orifice (22) et isole le troisième orifice (23) ; et une seconde configuration stable dans laquelle la chambre à orifice (40) fournit une communication fluidique entre le premier orifice (21) et le troisième orifice (23) et isole le deuxième orifice (22), dans lequel une surface externe (34) du noyau de vanne (27) est conçue pour se mettre en prise avec une surface interne (32) du corps de vanne (25) afin de définir un encliquetage permettant de résister à la rotation du noyau de vanne (27) entre la première configuration stable et la seconde configuration stable ; et un actionneur surmonte l'encliquetage afin de faire tourner le noyau de vanne (27) entre les première et seconde configurations stables ;
l'accouplement d'un tube respiratoire du patient au premier orifice (21) ;
l'accouplement d'un premier dispositif de ventilation au deuxième orifice (22) ;
pendant que la vanne (20) est dans la première configuration stable, l'accouplement d'un second dispositif de ventilation au troisième orifice (23) ;
le passage de la vanne (20) à la seconde configuration stable ; et
le débranchement du premier dispositif de ventilation du deuxième orifice (22).
